# EUROPEAN PATENT APPLICATION

(11) **EP 4 834 313 A1**
(43) Date of publication of application: **04.11.2026**
(21) Application number: 24912718.4
(22) Date of filing: 23.12.2024
(51) Int. Cl.: A61K 8/11, A61K 8/34, A61K 8/72

(54) **SOFT CAPSULE FOR COSMETIC USE**

(30) Priority: 28.12.2023 JP 2023222890
(71) Applicant: Morishita Jintan Co., Ltd., Osaka-shi, Osaka 540-8566 (JP)
(72) Inventor: KITA, Yusuke, Hirakata-shi, Osaka 573-0128 (JP); HASHIMOTO, Taku, Hirakata-shi, Osaka 573-0128 (JP); NISHIKAWA, Takehiro, Hirakata-shi, Osaka 573-0128 (JP)
(74) Representative: dompatent
(86) International application number: PCT/JP2024/045448
(87) International publication number: WO 2025/142831

(57) **Abstract**

An object of the present invention is to provide a capsule with which when used as a cosmetic, the capsule quickly disappears by grinding with fingers, and a satisfying sense of use is obtained. The present invention relates to a soft capsule for cosmetic use comprising a content that is liquid at room temperature and a shell that encapsulates the content, wherein shell pieces obtained by pulverizing the soft capsule with a bead type homogenizer show a particle size distribution in which a particle diameter having a volume-based cumulative frequency of 90% is 1000 µm or less.

## Description

### TECHNICAL FIELD

The present invention relates to a soft capsule used in cosmetics.

### BACKGROUND ART

Capsule formulations in which a drug substance is coated with a shell are used in various fields. For example, capsules are also used in the cosmetic field, and various capsules are used in embodiments, such as separating the capsule content from cosmetic components to improve storage stability; disintegrating the capsule at the time of use to release fresh content; or aiming at a dynamic design impact by using coloring of the capsule outer shell.

WO2018/043661 (Patent Document 1) proposes an agar shell capsule including: content made of an oily component; and an agar shell covering the content, wherein the agar shell is blended with silica. Provided is a capsule with which: when the capsule is used for a cosmetic containing a capsule to be applied onto the skin, the capsule is not crushed during storage; and when the capsule is applied onto the skin and then ground with fingers, the capsule is easily crushed, and thereby gel residues of the capsule shell do not remain on the skin or the gel residues of the capsule shell are reduced in amount. Patent Document 1 describes that silica added to an agar shell is a component that smooths the skin when the agar shell is ground on the skin of the user, and imparts a cleansing force to the cosmetic.

### PRIOR ART DOCUMENT

### PATENT DOCUMENT

Patent Document 1: WO2018/043661

### SUMMARY OF THE INVENTION

### PROBLEMS TO BE SOLVED BY THE INVENTION

When a cosmetic is used, the user uses it with a very high sense of fingers. Therefore, there is a problem that even the content of the conventional techniques does not necessarily achieve sense of use with which the user is satisfied enough, and sense of disappearance of the capsule shell that satisfies the user. An object of the present invention is to solve this. That is, an object of the present invention is to provide a capsule with which even when the capsule is used as a cosmetic and ground with fingers to produce shell pieces, the presence of the shell pieces is hardly felt with fingers, and the content is easily fitted to the skin.

### SOLUTIONS TO THE PROBLEMS

The present inventors intensively studied to solve the above object, and found that the above object is achieved when the capsule has a particle size distribution in which the particle diameter having a volume-based cumulative frequency of 90% is 1000 µm or less. Thereby, the present invention has been completed.

The present invention includes and provides the following embodiment.
[1] A soft capsule for cosmetic use comprising a content that is liquid at room temperature and a shell that encapsulates the content,
   wherein shell pieces obtained by pulverizing the soft capsule with a bead type homogenizer show a particle size distribution in which a particle diameter having a volume-based cumulative frequency of 90% is 1000 µm or less.

### EFFECTS OF THE INVENTION

The present invention makes it possible that after the soft capsule is crushed on the skin, the presence of the shell pieces is hardly felt with hands, and the content is easily fitted to the skin, when shell pieces obtained by pulverizing the soft capsule for cosmetic use with a bead type homogenizer have a particle size distribution in which the particle diameter having a volume-based cumulative frequency of 90% is 1000 µm or less.

### BRIEF DESCRIPTION OF THE DRAWINGS

[Fig. 1] Fig. 1 is a schematic vertical sectional view showing one embodiment of a nozzle unit of a device to produce the soft capsule for cosmetic use of the present invention.
[Fig. 2] Fig. 2 is a chart for explaining the requirement "a particle diameter having a volume-based cumulative frequency of 90% is 1000 µm or less" in the particle size distribution, and showing a case where the relationship between the relative particle amount (volume%) and the particle diameter (µm) is a normal distribution in the particle size distribution.
[Fig. 3] Fig. 3 is a chart showing a graph in which the volume-based particle size distribution measured in Examples 1 to 5 and Comparative Examples 1 to 2 is shown such that the vertical axis represents the obtained relative particle amount (volume%) and the horizontal axis represents the particle diameter (µm).
[Fig. 4] Fig. 4 is a chart showing a graph in which the volume-based particle size distribution measured in Examples 6 to 8 is shown such that the vertical axis represents the obtained relative particle amount (volume%) and the horizontal axis represents the particle diameter (µm).

### DETAILED DESCRIPTION

The present invention provides a soft capsule for cosmetic use comprising a content that is liquid at room temperature and a shell that encapsulates the content. The soft capsule achieves the technical effect described above when shell pieces obtained by pulverizing the soft capsule with a bead type homogenizer have a particle size distribution in which the particle diameter having a volume-based cumulative frequency of 90% is 1000 µm or less.

### Content

The soft capsule is used in cosmetics, and comprises: a content that is liquid at room temperature and a shell that encapsulates the content. The soft capsule content usually contains an oily liquid. The oily liquid may be a naturally derived liquid, a liquid obtained by synthesis, or a mixture thereof. Such an oily liquid is, for example, at least one selected from the group consisting of avocado oil, linseed oil, almond oil, olive oil, cacao oil, beef tallow, tung oil, wheat germ oil, sesame oil, rice germ oil, rice bran oil, safflower oil, soybean oil, evening primrose oil, camellia oil, corn oil, rapeseed oil, horse fat, persic oil, palm oil, palm kernel oil, castor oil, sunflower oil, pig fat, grape oil, jojoba oil, macadamia nut oil, mink oil, cottonseed oil, Japan wax, coconut oil, peanut oil, lanolin, egg yolk oil, rose hip oil, and medium chain fatty acid triglyceride, but is not limited thereto, and any component can be used.

The content may contain a beauty component. The beauty component may be any component as long as it is a component for the purpose of beauty or skin care that is generally contained in cosmetics, and examples thereof include vitamin components such as vitamin C and vitamin E; moisturizing components such as hyaluronic acid, ceramide, and collagen; and whitening components such as tranexamic acid and arbutin. The beauty component is not limited to the above, and any component can be used, and one or two or more beauty components can be used. The beauty component can be blended to an oily liquid that is the content; is modified so that it can be blended thereto; or used with another component so that it can be blended to an oily liquid. For example, an aqueous component or a hydrophilic component may be blended in an oily liquid using a surfactant.

In the soft capsule of the present invention, the amount of the beauty component is usually 1 to 50 wt%, preferably 5 to 30 wt%, and more preferably 10 to 20 wt%, based on the total amount of the content of the soft capsule. When the amount is more than 50 wt%, encapsulation may be difficult; and when the amount is less than 1 wt%, no effect of the active substance may be exhibited.

The content of the soft capsule of the present invention may be further appropriately blended with a blending agent such as an excipient, a stabilizer, an auxiliary agent, or a foaming agent. The amount of these blending agents is not particularly limited as long as the function of the soft capsule of the present invention is not inhibited. The content of the soft capsule of the present invention can also contain silica. Silica is said to improve tension and gloss of the skin.

### Shell

The shell of the soft capsule of the present invention makes it possible that: the content can be encapsulated; the soft capsule can be crushed with hands on the skin; and when the shell is being ground with fingers after crushed, the presence of the shell pieces is hardly felt or not felt with hands. The shell of the soft capsule usually contains a water-soluble polymer, a polyol, and water. When a polyol is contained, the shell pieces are easily made finer.

The water-soluble polymer is a component that forms the shell, and is selected from, for example, gelatin, casein, zein, pectin or a derivative thereof, alginic acid or a salt thereof, agar, gellan gum, carrageenan, furcellaran, chitosan, curdlan, starch, modified starch, pullulan, mannan, and mixtures thereof. Among them, the water-soluble polymer is preferably agar, and more preferably an agar having a jelly strength of 400 g/cm² or more. When the water-soluble polymer is agar, the presence of the shell pieces is hardly felt with hands, and the content is easily fitted to the skin after the soft capsule is crushed on the skin. When the water-soluble polymer is an agar having a jelly strength of 400 g/cm² or more, shape retainability as a capsule is easily obtained. The upper limit of the jelly strength of the agar is not particularly limited, but the jelly strength may be 2500 g/cm² or less from the viewpoint of availability. Specific examples of the jelly strength are 400 g/cm², 500 g/cm², 600 g/cm², 700 g/cm², 800 g/cm², 900 g/cm², 1000 g/cm², 1300 g/cm², 1500 g/cm², 1800 g/cm², 2000 g/cm², and 2500 g/cm², and may be within a range between any two of these numerical values. When the capsule is hardly crushed during use, the jelly strength can be 1300 g/cm² or less, for example.

The water-soluble polymer used in the present invention is not limited thereto. These water-soluble polymers are contained in an amount of 5 to 20 wt%, preferably 15 wt% or less, based on the total solid content weight of the shell of the soft capsule. When alginate (in particular, sodium alginate), gellan gum, pectin, or carrageenan is used as a curing aid or a viscosity modifier, an alkali metal salt, an alkaline earth metal salt, an ammonium salt, or the like may be appropriately added.

The shell contains water as described above. The water is a component with which the soft capsule becomes a wet state. For example, the water is at least one selected from the group consisting of tap water, ion-exchanged water, distilled water, and ultrapure water. The amount of the water is 60 wt% or more based on the total weight of the shell.

The polyol to be blended in the shell of the soft capsule of the present invention, having a plurality of hydroxyl groups in one molecule, is also called a polyhydric alcohol. The polyol is a water-soluble compound. The polyol has a molecular weight of 1000 or less, preferably 500 or less. The lower limit of the molecular weight of the polyol is not particularly limited, but the molecular weight of the polyol may be 90 or more. The polyol is, for example, at least one selected from the group consisting of butylene glycol, 1,2-propanediol, 1,3-propanediol, dipropylene glycol, sorbitol, glucose, trehalose, and polyethylene glycol, but is not limited to the above, and any component can be used.

The blending amount of the polyol in the shell is within a range of 5 to 10 parts by weight when the weight of the shell is 100 parts by weight. When the amount of the polyol is within the above range, the presence of the shell pieces is hardly felt with hands, and the content is easily fitted to the skin after the soft capsule is crushed on the skin. The blending amount of the polyol in the shell is preferably 40 wt% or more based on the total solid content weight of the shell of the soft capsule. In this case, the upper limit of the blending amount of the polyol is not particularly limited as long as the soft capsule can be formed, but the blending amount of the polyol may be, for example, 95 wt% or less. Specific examples of the blending amount of the polyol are 40 wt%, 45 wt%, 50 wt%, 55 wt%, 60 wt%, 65 wt%, 70 wt%, 75 wt%, 80 wt%, 83 wt%, 85 wt%, 90 wt%, 92 wt%, and 95 wt%, and may be within a range between any two of these numerical values. When the blending amount of the polyol is 40 wt% or more, not only the shell can be provided with such flexibility that the soft capsule is easily crushed during use, but also residues can be less likely to remain when the soft capsule is ground with fingers. When the shell becomes too soft, for example, the blending amount of the polyol can be 90 wt% or less. In one embodiment of the present invention, the blending amount of the polyol is preferably 2 times or more the blending amount of the water-soluble polymer. The blending amount of the polyol may be 3 times or more, 5 times or more, 7 times or more, or 9 times or more the blending amount of the water-soluble polymer, in terms of mass ratio. When the blending amount of the polyol is 2 times or more the blending amount of the water-soluble polymer, the presence of the shell pieces is hardly felt with hands, and the content is easily fitted to the skin after the soft capsule is crushed on the skin. The upper limit of the blending amount of the polyol is not particularly limited as long as the shell can be formed, but is, for example, 20 times or less. The blending amount of the polyol may be 17 times or less, 15 times or less, 12 times or less, or 10 times or less the blending amount of the water-soluble polymer.

If necessary, the shell of the soft capsule of the present invention may contain various additives commonly used in this field, such as fragrances, sweeteners, colorants, and preservatives such as parabens, in addition to the shell composition described above. When such an additive is used, the total content of all the additives is, for example, 0.01 to 10 wt%, preferably 0.1 to 5 wt% based on the total solid content weight in the composition to be the shell of the capsule. Note that, in the present invention, the shell of the soft capsule may contain no poorly water-soluble particles. The poorly water-soluble particles are particles constituted by a substance that is hardly dissolved in water. Examples of the substance constituting the poorly water-soluble particles include: inorganic substances such as zirconia-based and silica-based substances; and organic substances such as melamine-based and acryl-based substances. When the shell contains poorly water-soluble particles, the poorly water-soluble particles may remain on the skin so that discomfort such as powdery feeling occurs, after the shell is ground. In particular, when the soft capsule is a seamless capsule, during the shell liquid passing through a multiple nozzle, as described later, the multiple nozzle is easily clogged with the poorly water-soluble particles, and it may be difficult to form the seamless capsule.

The shell of the soft capsule of the present invention desirably has a thickness of 10 to 1000 µm, preferably 30 to 500 µm, and more preferably 50 to 250 µm. When the thickness of the shell is less than 10 µm, the shell strength tends to be low; and when the thickness exceeds 1000 µm, the content amount decreases, and the shell is poorly crushed.

The size of the soft capsule of the present invention is not particularly limited, but having a diameter of 0.3 to 10 mm, preferably 1 to 8 mm is desirable. When the diameter of the capsule is less than 0.3 mm, there is a tendency that less amount of components can be blended in the content; and when the diameter exceeds 10 mm, it takes time to grind the capsule.

Since it is assumed that the soft capsule of the present invention is used in a state where the soft capsule is dispersed in a continuous phase containing water as a main component, the capsule is preferably in a wet state. In such a capsule in a wet state, the shell preferably contains water in an amount of 60 wt% or more based on the total weight thereof. Note that the continuous phase refers to the phase outside the soft capsule in a state where the soft capsule is present in the continuous phase, and refers to the main body of a cosmetic preparation (preparation other than the soft capsule).

### Another layer

The soft capsule of the present invention basically includes two layers: the content and the shell, but one or more intermediate layers may be provided between the content and the shell as necessary or depending on the type of the content. It is also conceivable that the intermediate layer is present to prevent the content from adversely affecting the shell or inhibit the solubility of the content.

An intermediate layer contains fats and oils having a melting point of 45°C or higher. As the fats and oils having a melting point of 45°C or higher, those having a melting point of 45°C or higher are selected among the fats and oils having a melting point of 40°C or higher as described above. Specific examples of the fats and oils having a melting point of 45°C or higher include the above-mentioned plant (fractionated) fats and oils, beeswax, highly hydrogenated oil, margarine, and shortening.

The intermediate layer may be blended with lecithin or silicon dioxide in order to adjust interfacial tension, viscosity, and specific gravity. The amount of these blending agents is not particularly limited as long as the function of the soft capsule of the present invention is not inhibited.

### Production method of soft capsule

The soft capsule of the present invention may be produced by any method. In the following, the production method of the soft capsule will be described with reference to a case where the soft capsule is a seamless capsule.

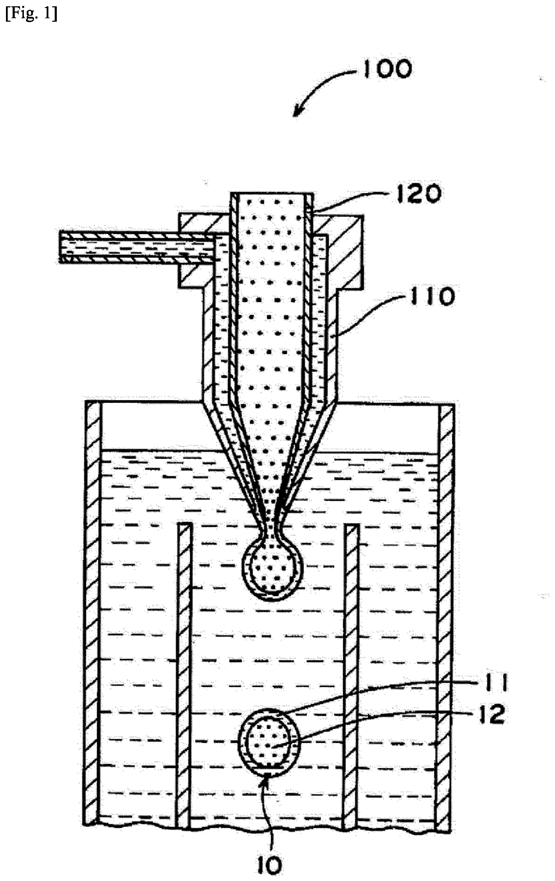

For the soft capsule of the present invention, examples thereof usually include a dropping method using a multiple nozzle, especially, a method of dropping into a cooling liquid by using a two-layer nozzle (double nozzle). Fig. 1 is a schematic vertical sectional view showing one embodiment of a nozzle unit of a device to produce the soft capsule (that is, seamless capsule) of the present invention.

In Fig. 1, the production device of the soft capsule uses a dropping method using a double nozzle 100. In the double nozzle 100, a content liquid is discharged from an inner nozzle 120, and a shell liquid is discharged from an outer nozzle 110. When an intermediate layer is included between the shell and the content, another nozzle may be added so that the soft capsule is produced by using a three-layer nozzle including an intermediate nozzle. During discharge, the liquids are pushed out at the same time and at a constant speed into a cooling liquid flowing down at a constant speed to form a complex jet, and released into the cooling liquid. Thereby, surface tension acts between the cooling liquid and the shell liquid, so that a soft capsule 10 can be continuously produced. In Fig. 1, the soft capsule 10 is constituted by a shell 11 and content 12.

When the soft capsule of the present invention is produced by using a two-layer nozzle as shown in Fig. 1, the obtained capsule has a two-layer structure. When a three-layer nozzle (not shown) is used, the obtained capsule has a three-layer structure. In both of the two-layer capsule and the three-layer capsule, the content is present as their innermost layer.

Immediately after the soft capsule is prepared in a cooling liquid as described above, the cooling liquid adheres to the outer surface of the soft capsule. Therefore, the production method of the soft capsule preferably includes a separation step of separating the soft capsule and the cooling liquid. The production method does not necessarily include a drying step.

### Particle size distribution of shell pieces of soft capsule

The soft capsule obtained by the above method requires that shell pieces obtained by pulverizing the soft capsule with a bead type homogenizer have a particle size distribution in which the particle diameter having a volume-based cumulative frequency of 90% is 1000 µm or less, as described above. Thereby, when the soft capsule is blended into cosmetics, the presence of the shell pieces is hardly felt when the soft capsule is ground with fingers during use, and the content is easily fitted to the skin. In other words, the soft capsule has a shell whose particle diameter having a volume-based cumulative frequency of 90% is 1000 µm or less, so that when the soft capsule is ground with fingers for use, the shell easily becomes small pieces.
Therefore, the presence of the shell pieces is hardly felt during use, and the content is easily fitted to the skin. The particle size distribution of the shell pieces described above is a physical property value of the shell indicating how easily the shell becomes small pieces, and does not necessarily coincide with the particle size distribution of the shell pieces obtained by grinding the shell on the skin during use.

When the soft capsule is pulverized with a bead type homogenizer, the soft capsule and the beads coexist in the tube of the homogenizer. Then, the bead type homogenizer applies vibration to cause the beads to collide with the soft capsule, and thereby the soft capsule can be pulverized. The pulverizing conditions of the soft capsule include a frequency of 18.0 Hz and a pulverization time of 90 seconds.

As long as the beads can pulverize the soft capsule, the material and particle diameter of each bead are not particularly limited. Examples of the material of the bead include ceramics, glass, and metal such as stainless steel. The particle diameter of the bead may be larger than the particle diameter of the soft capsule. When the bead is larger than the soft capsule, the particle diameter of the bead can be, for example, 4 to 5 times the particle diameter of the soft capsule. As a specific example, when the particle diameter of the soft capsule is 1.5 mm, the particle diameter of the bead can be 6.3 to 7.8 mm.

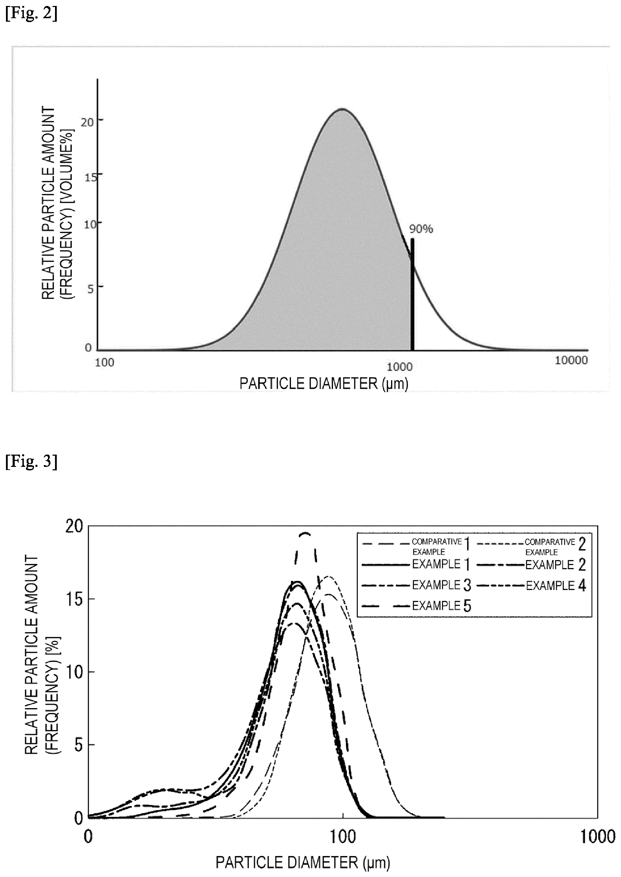

As described above, the present invention requires that shell pieces have a particle size distribution in which the particle diameter having a volume-based cumulative frequency of 90% is 1000 µm or less. In general, the particle size distribution is represented by a graph in which the horizontal axis represents the particle diameter (µm) and the vertical axis represents the particle amount (frequency) (volume%), and the particle diameter at the point where the cumulative volume of the curve reaches 90% of the total is the "particle diameter having a volume-based cumulative frequency of 90%". For example, Fig. 2 shows a case where the vertical axis represents the relative particle amount (frequency) in terms of volume% and the horizontal axis represents the particle diameter, and they are assumed to show a normal distribution for explanation. In Fig. 2, the "particle diameter having a volume-based cumulative frequency of 90%" can be determined when the curve is accumulated from a position where the particle diameter is smaller, and the particle diameter where volume% reaches 90% is specified. The present invention requires that the particle diameter having a volume-based cumulative frequency of 90% is 1000 µm or less (that is, 1 mm or less). When the particle diameter having a volume-based cumulative frequency of 90% is more than 1000 µm, the shell is less likely to become small pieces, and therefore a feeling of residue remains for a long time even when the shell is ground with fingers. The particle diameter having a volume-based cumulative frequency of 90% is preferably 960 µm or less. The particle diameter having a volume-based cumulative frequency of 90% may be 950 µm or less, 940 µm or less, 930 µm or less, 920 µm or less, 910 µm or less, or 900 µm or less. The particle diameter does not become 0 µm, but a smaller particle diameter is better, and about 100 µm is sufficient. The particle diameter having a volume-based cumulative frequency of 90% may be 200 µm or more, 300 µm or more, 400 µm or more, 500 µm or more, 600 µm or more, 700 µm or more, 800 µm or more, 850 µm or more, or 890 µm or more.

In the above, description has been made where the soft capsule is a seamless capsule, but the soft capsule is not limited to a seamless capsule, and may be a capsule obtained by a rotary method. When a rotary method is employed in the production of the soft capsule, the production device and the conditions of the production device can be arbitrarily selected.

### [Examples]

The present invention will be described in more detail with reference to examples. The present invention should not be understood to be limited to these examples. In the examples, the terms "parts", "%", and the like are all based on weight or weight.

### Example 1

Mixed were 1.3 parts by weight of agar A (agar having a jelly strength of 610 to 650 g/cm²), 0.6 parts by weight of sodium alginate, 10.0 parts by weight of 1,3-butylene glycol, and 88.1 parts by weight of ion-exchanged water, and the mixture was heated and dissolved at a temperature of 105°C for 10 minutes in an autoclave to obtain a shell liquid.

The shell liquid and medium chain fatty acid triglyceride as the content liquid were charged into a seamless capsule production device having a concentric double nozzle; the content liquid was charged in the inner nozzle of the double nozzle and the shell liquid was charged in the outer nozzle thereof; the temperature of the content liquid was 20°C and the temperature of the shell liquid was 70°C; and they were discharged into flowing medium chain fatty acid triglyceride (10°C) to produce a seamless capsule having a particle diameter of 1.5 mm.

After the obtained seamless capsule was degreased, 75 parts by weight of the seamless capsule was immersed in 25 parts by weight of a continuous phase (mixture of 4.0 parts by weight of 1,3-butylene glycol, 1.0 parts by weight of glycerin, 0.1 parts by weight of phenoxyethanol, and 94.9 parts by weight of ion-exchanged water) to prepare a cosmetic.

### Pulverization of seamless capsule and measurement of particle size distribution

For the seamless capsule, the capsule taken out from the cosmetic obtained in examples was pulverized by the following procedure to obtain a pulverized product of the shell. Then, the particle size distribution of the pulverized product of the shell was measured, and the particle diameter having a volume-based cumulative frequency of 90% was determined as follows.

Two glass beads (SANSYO glass bead No. 7) (about 0.9 g) and 0.2 g of the seamless capsule were put into a microtube (specifically, 2 mL microtube (AS ONE GDMST-2ML)) of a bead type homogenizer (TissueLyser II manufactured by QIAGEN), and then covered, which was set in the bead type homogenizer, and the seamless capsule was pulverized under the conditions of a frequency of 18.0 Hz and a time of 90 seconds. After pulverization, the shell pieces were washed once with 0.5 mL of ethanol and twice with 0.5 mL of hexane to obtain a shell piece sample, which was repeated to obtain 3 g of a shell piece sample.

The obtained shell piece sample was measured for volume-based particle size distribution and average diameter (or particle diameter having a cumulative frequency of 90%) under a wet condition in ethanol by using a laser diffraction scattering type particle size distribution analyzer (SALD-2300 manufactured by Shimadzu Corporation). The volume-based particle size distribution is described in Fig. 3, where the measurement values are shown in a graph in which the vertical axis represents the relative particle amount (volume%) and the horizontal axis represents the particle diameter (µm). The obtained capsule was subjected to the following sensory test.

### <Sensory evaluation>

0.1 g of the capsule was placed on the back of the hand, and the capsule and the shell pieces were ground so as to draw a circle with the ball of the finger of the opposite hand, which was subjected to sensory evaluation in accordance with the following criteria.
-: It takes 30 seconds or more for breaking all the capsules, the size of the remaining shell pieces is larger than the particle diameter (1.5 mm), the presence of the shell pieces is felt with hands, and therefore there is no improvement in the fitness feeling of the content to the skin.
+: It takes 15 to 30 seconds for breaking all the capsules, the size of the remaining shell pieces is equal to or lower than the particle diameter (1.5 mm), the presence of the shell pieces is hardly felt with hands, and there is an improvement in the fitness feeling of the content to the skin.
++: It takes 15 seconds or less for breaking all the capsules, the size of the remaining shell pieces is equal to or lower than the particle diameter (1.5 mm), the presence of the shell pieces is hardly felt with hands, and there is an improvement in the fitness feeling of the content to the skin.

The results of the sensory evaluation using capsules are shown in Table 1. Table 1 also lists the formulation of the shell, the mass ratio of polyol/water-soluble polymer, the particle diameter having a volume-based cumulative frequency of 90%, and the shell thickness (µm).

### Examples 2 to 5 and Comparative Examples 1 to 2

In accordance with the formulation shown in Table 1, a shell liquid of the capsule was prepared, and a seamless capsule and a cosmetic were prepared in the same manner as in Example 1. In the same manner as in Example 1, the particle diameter having a volume-based cumulative frequency of 90% was measured and sensory evaluation was performed, and the results are described in Table 1. Agar B described in Table 1 is an agar having a jelly strength of 900 to 1000 g/cm². In Table 1, Na alginate is sodium alginate, 1,3-BG is 1,3-butanediol, and PG is 1,2-propanediol. Further, in the same manner as in Example 1, the particle size distribution of each of the examples and comparative examples is shown in a graph in which the vertical axis represents the relative particle amount (volume%) and the horizontal axis represents the particle diameter (µm), which is described in Fig. 3.

### Examples 6 to 8

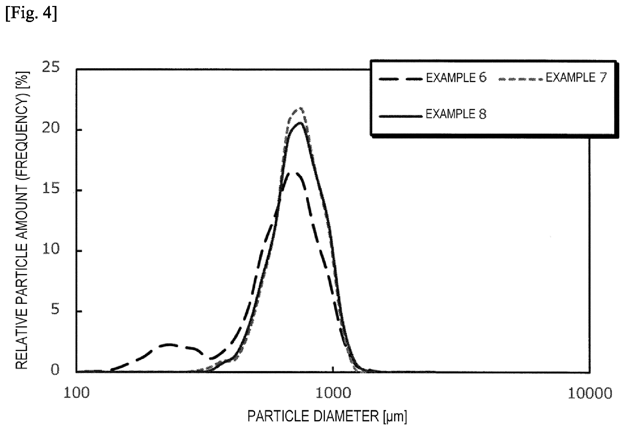

In accordance with the formulation shown in Table 2, a shell liquid of the capsule was prepared, and a seamless capsule and a cosmetic were prepared in the same manner as in Example 1. In the same manner as in Example 1, the particle diameter having a volume-based cumulative frequency of 90% was measured and sensory evaluation was performed, and the results are described in Table 2. Table 2 also lists the mass ratio of polyol/water-soluble polymer and the shell thickness in the same manner as Table 1. Agar C described in Table 2 is an agar having a jelly strength of 1800 g/cm². In the same manner as in Example 1, the particle size distribution of each of the examples is shown in a graph in which the vertical axis represents the relative particle amount (volume%) and the horizontal axis represents the particle diameter (µm), which is described in Fig. 4.

The jelly strength was measured by a method in accordance with Nikkansui method. Specifically, the jelly strength means a maximum weight per 1cm² that a cooled jelly withstands for 20 seconds, where the cooled jelly is obtained after a 1.5 wt% solution of agar is boiled and dissolved, and left to stand at 20°C for 15 hours.

**[Table 1]**

| | Example 1 | | Example 2 | | Example 3 | | Example 4 | | Example 5 | | Comparative Example 1 | | Comparative Example 2 | |
|---|---|---|---|---|---|---|---|---|---|---|---|---|---|---|
| Shell formulation [wt%] | Agar A | 1.33 | Agar A | 1.33 | Agar A | 1.33 | Agar A | 1.33 | Agar B | 1.11 | Agar B | 1.11 | Agar A | 1.33 |
| | Na alginate | 0.63 | Na alginate | 0.63 | Na alginate | 0.63 | Na alginate | 0.63 | Na alginate | 0.52 | Na alginate | 0.52 | Na alginate | 0.63 |
| | 1,3-BG | 10.00 | 1,3-BG | 5.00 | PG | 10.00 | Sorbitol | 10.00 | 1,3-BG | 10.00 | | | | |
| | Ion-exchanged water | 88.04 | Ion-exchanged water | 93.04 | Ion-exchanged water | 88.04 | Ion-exchanged water | 88.04 | Ion-exchanged water | 88.37 | Ion-exchanged water | 98.37 | Ion-exchanged water | 98.04 |
| Polyol/Water-soluble polymer | 5.1 | | 2.6 | | 5.1 | | 5.1 | | 6.1 | | 0 | | 0 | |
| Sensory evaluation | ++ | | ++ | | ++ | | + | | ++ | | - | | - | |
| Particle diameter having volume-based cumulative frequency of 90% [µm] | 939 | | 952 | | 929 | | 938 | | 892 | | 1,384 | | 1,370 | |
| Shell thickness [µm] | 143 | | 139 | | 138 | | 139 | | 138 | | 139 | | 140 | |

**[Table 2]**

| | Example 6 | | Example 7 | | Example 8 | |
|---|---|---|---|---|---|---|
| Shell formulation [wt%] | Agar A | 0.70 | Agar A | 1.70 | Agar C | 1.00 |
| | Na alginate | 0.33 | Na alginate | 0.80 | Na alginate | 0.63 |
| | 1,3-BG | 10.00 | 1,3-BG | 5.00 | 1,3-BG | 10.00 |
| | Ion-exchanged water | 88.97 | Ion-exchanged water | 92.50 | Ion-exchanged water | 88.37 |
| Polyol/Water-soluble polymer | 9.7 | | 2.0 | | 6.1 | |
| Sensory evaluation | ++ | | ++ | | ++ | |
| Particle diameter having volume-based cumulative frequency of 90% [µm] | 894 | | 918 | | 928 | |
| Shell thickness [µm] | 138 | | 139 | | 139 | |

In Examples 1 to 8, where the particle diameter of shell pieces having a volume-based cumulative frequency of 90% was 1000 µm or less in the particle size distribution, the sensory test performed when the capsule was ground all resulted in a sensory evaluation of + or ++, and the capsule was broken early and the presence of the shell pieces was hardly felt with hands. As for the mass ratio of polyol/water-soluble polymer, excellent performance is exhibited when the blending amount of polyol is larger than that of water-soluble polymer. On the other hand, in Comparative Examples 1 and 2, where the particle diameter of shell pieces having a volume-based cumulative frequency of 90% was more than 1000 µm in the particle size distribution, the sensory test resulted in an evaluation of "-", and the capsule was crushed in 30 seconds or more, the size of the shell pieces (residue) was large, and a good sense of use could not be obtained. The comparative examples are examples in which no polyol is present.

### INDUSTRIAL APPLICABILITY

When the soft capsule of the present invention is used in cosmetics, the capsule is ground in a short time during use; no shell pieces are present; and the user feels an excellent sense of use. The soft capsule of the present invention can be used as follows: a component having a function other than that of a cosmetic or having the same but improved function is mixed in a cosmetic; the capsule is picked up in hand during use and ground with fingers; and the functional component is released, thereby successfully improving the function of cosmetics. The soft capsule of the present invention may also be colored, and the color of cosmetics can be adjusted by the capsule.

### [Embodiments of invention]

[1] A soft capsule for cosmetic use comprising a content that is liquid at room temperature and a shell that encapsulates the content,
   wherein, shell pieces obtained by pulverizing the soft capsule with a bead type homogenizer show a particle size distribution in which a particle diameter having a volume-based cumulative frequency of 90% is 1000 µm or less.
[2] The soft capsule for cosmetic use according to [1], wherein the content is an oily liquid.
[3] The soft capsule for cosmetic use according to [1] or [2], wherein the shell contains a water-soluble polymer and a polyol.
[4] The soft capsule for cosmetic use according to any one of [1] to [3], wherein the polyol has an amount within a range of 5 to 10 parts by weight when the shell has a weight of 100 parts by weight.
[5] The soft capsule for cosmetic use according to any one of [1] to [4], wherein the polyol is at least one component selected from the group consisting of butylene glycol, 1,2-propanediol, 1,3-propanediol, dipropylene glycol, sorbitol, glucose, trehalose, and polyethylene glycol.
[6] The soft capsule for cosmetic use according to any one of [1] to [5], wherein, in the soft capsule, the shell has a water content of 60 wt% or more.
[7] The soft capsule for cosmetic use according to any one of [1] to [6], wherein the soft capsule is a seamless capsule.
[8] The soft capsule for cosmetic use according to any one of [1] to [7],
   wherein in the pulverization with the bead type homogenizer,
   a frequency is 18.0 Hz and a pulverization time is 90 seconds,
   a bead for pulverizing the soft capsule is a glass bead, and
   a particle diameter of the glass bead is larger than a particle diameter of the soft capsule.

### REFERENCE SIGNS LIST

- 10: soft capsule
- 11: shell
- 12: content
- 100: double nozzle
- 110: outer nozzle
- 120: inner nozzle

## Claims

1. A soft capsule for cosmetic use comprising a content that is liquid at room temperature and a shell that encapsulates the content,
wherein shell pieces obtained by pulverizing the soft capsule with a bead type homogenizer show a particle size distribution in which a particle diameter having a volume-based cumulative frequency of 90% is 1000 µm or less.

2. The soft capsule for cosmetic use according to claim 1, wherein the content is an oily liquid.

3. The soft capsule for cosmetic use according to claim 1 or 2, wherein the shell contains a water-soluble polymer and a polyol.

4. The soft capsule for cosmetic use according to claim 3, wherein the polyol has an amount within a range of 5 to 10 parts by weight when the shell has a weight of 100 parts by weight.

5. The soft capsule for cosmetic use according to claim 3 or 4, wherein the polyol is at least one component selected from the group consisting of butylene glycol, 1,2-propanediol, 1,3-propanediol, dipropylene glycol, sorbitol, glucose, trehalose, and polyethylene glycol.

6. The soft capsule for cosmetic use according to any one of claims 1 to 5, wherein, in the soft capsule, the shell has a water content of 60 wt% or more.

7. The soft capsule for cosmetic use according to any one of claims 1 to 6, wherein the soft capsule is a seamless capsule.

8. The soft capsule for cosmetic use according to any one of claims 1 to 7,
wherein
in the pulverization with the bead type homogenizer,
a frequency is 18.0 Hz and a pulverization time is 90 seconds,
a bead for pulverizing the soft capsule is a glass bead, and
a particle diameter of the glass bead is larger than a particle diameter of the soft capsule.
